(19) Europäisches Patentamt / European Patent Office / Office européen des brevets

(11) **EP 3 722 782 A1**

(12) **EUROPEAN PATENT APPLICATION**
published in accordance with Art. 153(4) EPC

(43) Date of publication:
**14.10.2020 Bulletin 2020/42**

(51) Int Cl.:
*G01N 15/06* (2006.01)

(21) Application number: **18885562.1**

(22) Date of filing: **30.11.2018**

(86) International application number:
**PCT/CN2018/118694**

(87) International publication number:
**WO 2019/109870 (13.06.2019 Gazette 2019/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.12.2017 CN 201711268483**

(71) Applicant: **Fatri United Testing & Control (Quanzhou) Technologies Co., Ltd.**
**Fujian 362012 (CN)**

(72) Inventors:
• NIE, Yongzhong
  Fujian 361008 (CN)
• ZHANG, Zhongping
  Fujian 361008 (CN)

(74) Representative: **Angerhausen, Christoph**
**Boehmert & Boehmert**
**Anwaltspartnerschaft mbB**
**Pettenkoferstrasse 22**
**80336 München (DE)**

(54) **MAGNETIC INDUCTION PARTICLE DETECTION DEVICE AND CONCENTRATION DETECTION METHOD**

(57) The disclosure provides a device for detecting magnetic induction particles and a concentration detection method. The device includes a signal detection system, a detection pipeline, an excitation coil, and a positive even number of induction coils, wherein the excitation coil is connected to a signal processing system and wound on the detection pipeline; the induction coils are connected to the signal processing system respectively and wound on the excitation coil successively, and each of the induction coils is wound on the excitation coil reversely from another induction coil directly adjacent to the induction coil. The device is convenient for manufacture and installation, and can improve detection precision. The method includes S1: obtaining an output signal of the signal detection system, to acquire a change of a voltage amplitude; and S2: detecting a concentration of metal particles according to the change of the voltage amplitude. The method can improve calculation accuracy. (Fig. 1)

Fig. 1

EP 3 722 782 A1

## Description

## TECHNICAL FIELD

The present disclosure relates to the field of detection equipment, particularly to a device for detecting magnetic induction particles, and further to a method for concentration detection using the device.

## BACKGROUND

Currently, there are multiple approaches for detecting metal particles, among which, it is common to utilize the electromagnetic induction principle to detect the metal particles. Specifically, a conventional device for detecting metal particles using the principle of electromagnetic induction often uses two reverse-wound exciting/excitation coils as an excitation source, to generate two magnetic fields of the same intensity and opposite directions. In the absence of a magnetic field disturbance, a net magnetic field at the middle of the two coils is zero; an induction coil for inducing a change in the magnetic field is wound at the middle, to induce a magnetic field disturbance caused by metal particles.

Although such device is capable of electromagnetic detection of metal particles, the device still has the following drawbacks.

(1) In order to establish a balance of the magnetic field and induce a magnetic field signal of the metal particles, two reverse excitation coils and one induction coil are needed. This design makes a length of the sensor longer, which is not conducive to actual design, manufacture and installation.

(2) Only one magnetic induction coil is used. When the magnetic field is balanced by the electromagnetic induction, magnetic field attenuation outside the excitation coil (exciting coil) is relatively obvious. When a magnetic field disturbance caused by small particles on the excitation coils, is reflected on the external induction coil, the magnetic field usually has been attenuated a lot. Therefore, a detection precision for tiny particles is insufficient, which affects a detection effect.

Further, a method for detecting using data measured by the prior art device is inaccurate correspondingly. It is difficult to detect concentration of metal particles in a fluid accurately.

## SUMMARY

In order to overcome deficiencies of the prior art, technical objects to be achieved by the present disclosure includes (1) providing a device for detecting magnetic induction particles, which can be manufactured and installed easily and is capable of improving detection precision, and (2) providing a method for concentration detection of metal particles using the device.

The present disclosure adopts the following technical solution to achieve the first technical object mentioned above.

A device for detecting magnetic induction particles includes a signal detection system, a detection pipeline, an excitation coil, and a positive even number of induction coils, wherein the excitation coil is connected to a signal processing system and wound on the detection pipeline; the induction coils are connected to the signal processing system respectively and wound on the excitation coil successively, and each of the induction coils is wound on the excitation coil reversely from another induction coil directly adjacent to the induction coil.

In the existing technical solutions of a device for detecting particles based on electromagnetic induction, the device usually needs two reverse exciting/excitation coils and one induction coil, which should be installed by a way that the excitation coils are wound outwardly and reversely at both ends of the pipeline and the induction coil is wound at the middle of the two excitation coils. In the present technical solution, the arrangement that induction coils are wound outside the excitation coil in the device, enables the device to be installed conveniently, and achieves an effect of greatly shortening the overall length of the sensor, such that it is convenient to be manufactured and installed.

The excitation coil is connected to the signal detection system. The signal detection system inputs a sinusoidal alternating signal to both ends of the excitation coil, to generate an alternating magnetic field which drives the induction coils. In addition, the induction coils are wound on the detection pipeline. As a result, a condition of the particles can be detected without contacting directly the liquid in the pipeline with a sensor, which makes the test more convenient.

In order to achieve an effect of improving the detection precision, the inventors employed a scheme of a positive even number of induction coils in the solution of the present disclosure. In the prior art, generally, only one magnetic induction coil is wound. Although using one magnetic induction coil seems to save costs, in fact, because the induction coil is arranged at the middle of the two excitation coils and the induction coil is relatively far from the excitation coils, when a magnetic field disturbance is caused by induction particles passing through the excitation coils, the magnetic field is tend to attenuate a lot, leading to insufficient precision for detecting the size of the induction particles.

In the present technical solution, the excitation coil is used and wound by a positive even number of induction coils, to ensure the detection precision. The excitation coil is used to generate a magnetic field, and thus preferably, one excitation coil is wound. A positive even number (for example, two) of induction coils, or a group of induction coils, are used, so as to adapt to the subsequent algorithm set by the inventors, i.e., calculating concentration of metal particles based on observing a change the magnetic field when the metal particles are input into and pass through the two induction coils.

The induction coils are wound on the excitation coil successively. Such arrangement can detect quickly a disturbance on the magnetic field generated when the particles pass through the induction coil, and achieve a function of detecting metal particles.

The induction coils are wound on the excitation coil reversely. The environment where the induction coils are located may be considered consistent, since the induction coils are close to each other. Such arrangement can suppress temperature drift and electromagnetic interference in a complex and harsh environment, improve signal stability, and further improve the system performance.

It is to be noted that the coil refers to a segment of coil that is connected to the signal detection system at both ends and wound around the detection pipeline.

It is to be noted that successive winding refers to that, for example, one of two induction coils is not to be wound until the other one has been wound, and the one induction coil will be wound at a position next to the other one in the winding direction. That is to say, the induction coils are wound on the detection pipeline in a way that each of the induction coils is not overlapped with each other but wound on the detection pipeline independently.

It is to be noted that reverse winding refers to that, when being wound, two induction coils are wound outside the excitation coil without overlap with each other, and one of the induction coils is wound clockwise and the other is wound counterclockwise. It is to be noted that, the detection of particles as used herein refers to detection of particles, such as, metal particles, by the electromagnetic induction, and detection of the flow thereof, so as to facilitating further analysis of data such as concentration of the metal particles in the liquid.

It is to be noted that the signal detection system is used for detecting electromagnetic induction conditions. In an optional embodiment, it includes a control circuit board, a signal output port, and the like. The signal detection system should not be limited to the composition as shown. Any mechanism should be regarded as belonging to the signal detection system, as long as it can detect the electromagnetic change of the induction coils.

It is to be noted that two adjacent, successively and reversely arranged induction coils constitute a group of induction coils.

Preferably, the number of induction coils is two or four or six.

In order to achieve an optimum balance between installation and manufacture costs and detection precision, it is preferable to set the number of induction coils as two.

Alternatively, by setting the number of induction coils as four or six, etc., it is possible to perform multiple measurements and average the measured values during the measurement process, which can improve the reliability of the detection.

Preferably, the excitation coils are two or more, and all the excitation coils are wound in the same direction on the detection pipeline respectively.

It is to be noted that winding in the same direction means that all the excitation coils are wound either clockwise or counterclockwise on the detection pipe respectively. By such arrangement, the magnetic field can be strengthened, and at the same time, winding in the same direction can prevent the mutual interferences between the excitation coils which affects the stability of the magnetic field.

Preferably, the excitation coil and/or the induction coils are wound are wound in a manner of at least one layer.

The excitation coil and/or the induction coils are wound in a manner of at least one layer (i.e., multiple layers), which can further enhance the strength of the magnetic field generated by the excitation coil, and the signal generated on the induction coils is more obvious, so as to facilitate improving the detection precision of metal particles.

Preferably, the detection pipeline is made of non-magnetic materials; and further preferably, the detection pipeline is made of stainless steel.

The pipeline is made of non-magnetic materials, so as to detect the magnetic field disturbance generated by the metal particles on the excitation coil more accurately. During the detection, it is necessary to ensure that the magnetic field generated by the excitation coil passes through the middle of the pipeline as possible, to increase the strength of the magnetic field in the pipeline. More preferably, the non-magnetic stainless steel meets the requirement, but is not limited to this material.

Preferably, a spacer sleeve is disposed between the excitation coil and the induction coils; and further preferably, the spacer sleeve is made of non-magnetic materials.

The spacer sleeve is added between the excitation coil and the induction coils, for isolating the excitation coil and the induction coils. The non-magnetic materials are selected here, mainly for isolation of the excitation coil and the induction coils during the production and manufacture process. During the process of inducing magnetic field disturbance generated by the metal particles, it is beneficial to improve the detection precision of metal particles by minimizing the magnetic field loss between the induction coils and the excitation coil, and thus the non-magnetic materials is used here. At the same time, the spacer sleeve plays a role of a skeleton for winding the induction coils, which may improve the flatness of the wound induction coils.

Preferably, a shielding ring is further disposed outside the induction coils.

The shielding ring arranged outside the induction coils may isolate an external magnetic field and resist the interference of the external magnetic field, so that a detection result is more accurate and a detection effect is better.

The present disclosure adopts the following technical solution to achieve the second technical object mentioned above.

A method for concentration detection using the device for detecting magnetic induction particles mentioned above includes the following steps:

> S1: obtaining an output signal of the signal detection system, to acquire a change of a voltage amplitude; and
> S2: detecting a concentration of metal particles according to the change of the voltage amplitude;

wherein the change of the voltage amplitude includes the change of the voltage amplitude in both an amplitude value and time, i.e., a relationship between a change of the amplitude value and a moment when it happens, for example, the amplitude value at a certain point and the corresponding moment; more specifically, it may be the highest point and zero point of the amplitude value and their corresponding moments.

Preferably, detecting the concentration of metal particles includes the following steps:

> obtaining a flow velocity (v) by which metal particles pass through the induction coils;
> obtaining a mass (m) of the metal particles; and calculating a concentration (c) of particles by the following equation, according to the obtained flow velocity (v) and mass (m) of the metal particles, and a time duration (t) took by the metal particles to pass through the induction coils, and utilizing a cross-sectional area (S) of the detection pipeline:

$$ c = \frac{m}{v * t * S} . $$

During the process of obtaining the mass (m) of the metal particles, in the single-layer closely wound coil, the induced voltage (E) caused by the metal particles when passing through the induction coils, is proportional to a volume (V), magnetic permeability, and passing velocity (v) of the particles, and the third power of a density of turns of the wound coils. The volume and mass of the metal particles flowing through the pipeline may be converted by quantitative analysis of the output signal of the oil sensor.

It is to be noted that the elapsed time t refers to the time required for the metal particles to pass through a certain distance in the pipeline, which may correspond to the time elapsed between different amplitude values, or a difference between moments of different amplitude values.

Preferably, obtaining the flow velocity (v) of the metal particles includes the following steps:

when the metal particles pass through a group of induction coils:

> recording first time and second time at which the signal processing system measures that the voltage amplitude is at the highest point and a zero-crossing point of a positive half cycle respectively, and calculating a first time difference (ΔT1) between the first time and the second time, and a first corresponding length (L1) of induction coil; recording third time and fourth time at which the signal processing system measures that the voltage amplitude is at a zero-crossing point and the highest point of a negative half cycle respectively, and calculating a second time difference (ΔT2) between the third time and the fourth time, and a second corresponding length (L2) of induction coil;
> measuring the flow velocity (v) based on the following equation:

$$ v = \frac{\dfrac{k1 * L1}{\Delta T1} + \dfrac{k2 * L2}{\Delta T2}}{2} . $$

It is to be noted that L1 refers to the length of induction coil passed through by the particles between the highest point and the zero-crossing point of the voltage amplitude during the positive half cycle; and the L2 refers to the length of induction coil passed through by the particles between the zero-crossing point and the highest point of the voltage amplitude during the negative half cycle.

k1 refers to a correction coefficient when the particles pass through one of the coils; and k2 refers to a correction coefficient when the particles pass through the other coil.

Because that different factors of each oil sensor, such as, the wire (thickness and material) the number of winding turns, the interaction of the two induction coils and so on, affect the output signal of the sensor, leading to that the sensor cannot sense the middle part of the coil, the correction coefficients k1 or k2 are used to correct it.

More specifically, during the process that ferromagnetic particles pass through the two induction coils, the ferromagnetic particles pass through the first induction coil and the second induction coil sequentially. When ferromagnetic particles pass through the first induction coil, the highest point of the output signal should be in the middle portion of the first induction coil, without consideration of an influence generated by the second induction coil. However, after the second induction coil is introduced, the magnetic field generated by the second induction coil would affect the highest point of the output signal to be slightly offset.

Preferably, if the induction coils includes multiple groups of induction coils, the flow velocity (v) by which the metal particles pass through the induction coils equals an average value of flow velocities (v) each corresponding to one of the groups of induction coils.

For example, in S1, the flow velocity (vgn) (where n is a positive integer) of the metal particles passing through the gn$^{th}$ group of induction coils is calculated respectively, and the flow velocity (v) is an average value of the flow velocity of each group of induction coils, that is:

$$v = \frac{vg1 + vg2 + \cdots + vgn}{n}$$

The calculation method of the average value can improve the calculation precision of the flow velocity, and the calculation result is more accurate.

Preferably, a frequency of obtaining the output signal of the signal detection system in S1 is once per microsecond ($\mu$s).

The beneficial effect of setting the obtaining frequency to be once per ms is that the frequency of the output signal is 500 Hz; according to the sampling theorem, the sampling frequency should be greater than 2 times of the highest frequency of the signal, in order to preserve complete information of the signal without distortion; and thus, the sampling frequency of 1K is selected here, i.e., 1000 valid signals are sampled in one second (one per ms) for analysis.

Compared with the prior art, the device for detecting magnetic induction particles of the present disclosure has the following beneficial effects.

1. The induction coils are wound outside the excitation coil in the present device, enabling the device to be installed conveniently, and achieving an effect of greatly shortening the overall length of the sensor, such that it is convenient to be manufactured and installed.

2. The induction coils are wound on the detection pipeline in the present device, such that a condition of the particles can be detected without contacting directly the liquid in the pipeline with the sensor, which makes the test more convenient.

3. At least two induction coils are used to wind the excitation coil in the present device, so as to ensure the detection precision.

4. The spacer sleeve is added between the excitation coil and the induction coils in the present device, for isolating the excitation coil and the induction coils and decreasing the magnetic field loss between the induction coils and the excitation coil; and at the same time, the spacer sleeve plays a role of a skeleton for winding the induction coils, which may improve the flatness of the wound induction coils.

5. In the present device, the shielding ring arranged outside the induction coils may isolate an external magnetic field and resist the interference of the external magnetic field, so that a detection result is more accurate and a detection effect is better.

The above description is only an overview of the technical solutions of the present disclosure. In order to illustrate the technical means of the present disclosure more clearly enabling it to be implemented according to the content of the specification, and make the above-described and other objects, features and advantages of the present disclosure to be more clear for understanding, the following preferred embodiments are provided, which may be detailed below in connection with the accompany drawings.

**BRIEF DESCRIPTION OF THE DRAWINGS**

Fig. 1 is a cross-sectional structural view showing a first preferred embodiment of a device for detecting magnetic induction particles of the present disclosure;

Fig. 2 is a cross-sectional structural view showing a second preferred embodiment of a device for detecting magnetic induction particles of the present disclosure;

Fig. 3 is a partial enlarged view of area A in Fig. 2;

Fig. 4 is a schematic diagram showing a principle for electromagnetic induction testing using the device for detecting magnetic induction particles of the present disclosure; and

Fig. 5 is a graph showing a voltage output change corresponding to the schematic diagram of Fig. 4.

**DETAILED DESCRIPTION**

In order to further illustrate the technical means and effects taken by the present disclosure to achieve the predetermined technical objects, specific embodiments, structures, features, and effects thereof according to the disclosure are described in details below with reference to the accompanying drawings and preferred embodiments.

**Embodiment I (Device for detecting magnetic induction particles)**

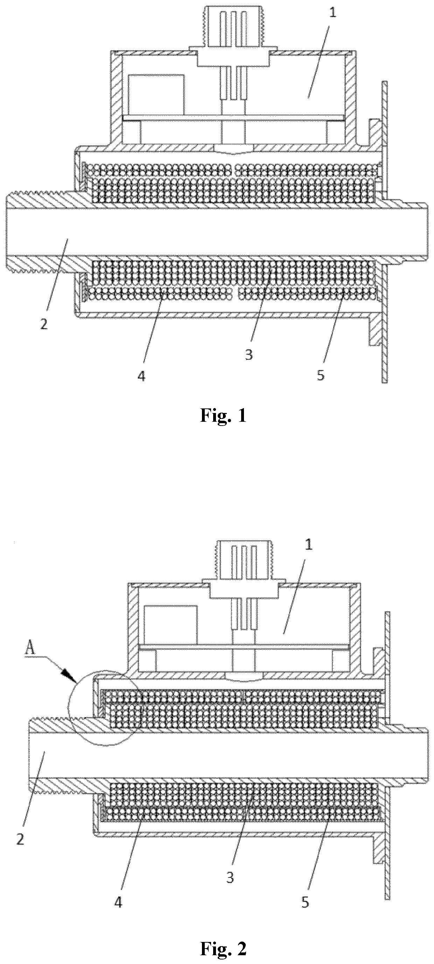

As shown, Fig. 1 is a cross-sectional structural view showing a first preferred embodiment of a device for detecting magnetic induction particles of the present disclosure. The device includes a signal detection system 1, a detection pipeline 2, an excitation coil 3, and two induction coils (a first induction coil 4 and a second induction coil 5, respectively). The excitation coil is connected to a signal processing system and wound on the detection pipeline. The induction coils are connected to the signal processing system respectively and wound on the excitation coil successively and reversely to each other.

The above is one of the preferred embodiments of the present technical solution. This basic implementation has the following beneficial effects.

(1) The arrangement that induction coils are wound outside the excitation coil in the device, enables the device to be installed conveniently, and achieves an effect of greatly shortening the overall length of the sensor, such that it is convenient to be manufactured and installed.

(2) The induction coils are wound on the detection pipeline, such that a condition of the particles can be detected without contacting directly the liquid in the pipeline with the sensor, which makes the test more convenient.

(3) The induction coils are wound on the excitation coil successively, which enables quick detection of the magnetic field disturbance generated when the particles pass through the induction coils, so as to achieve the function of detecting metal particles;

(4) The induction coils are wound on the excitation coil reversely. The environment where the induction coils are located may be considered consistent, since the induction coils are close to each other. Such arrangement can suppress temperature drift and electromagnetic interference in a complex and harsh environment, improve signal stability, and further improve the system performance.

In the embodiment, the number of the excitation coil for generating the magnetic field is one. In other embodiments, the number of excitation coils may also be two or more, but all the excitation coils should to be wound in the same direction, to prevent mutual interferences of magnetic fields which may affect the measurement effect.

In the embodiment, the number of the induction coils is two. By this arrangement, the detection precision may be improved effectively and the detection effect is better. Alternatively, in other embodiments, the number of the induction coils is a positive even number, such as, four, six or more. On the one hand, the same detection effect can be achieved, and on the other hand, the reliability of the detection can be improved by performing multiple measurements and averaging the measured values.

In this embodiment, the detection pipeline is made of non-magnetic materials; more specifically, the detection pipeline is made of stainless steel. The pipeline is made of non-magnetic materials, so as to detect the magnetic field disturbance generated by the metal particles on the excitation coil more accurately. During the detection, it is necessary to ensure that the magnetic field generated by the excitation coil passes through the middle of the pipeline as possible, to increase the strength of the magnetic field in the pipeline. More preferably, the non-magnetic stainless steel meets the requirement, but is not limited to this material.

**Embodiment II (Device for detecting magnetic induction particles)**

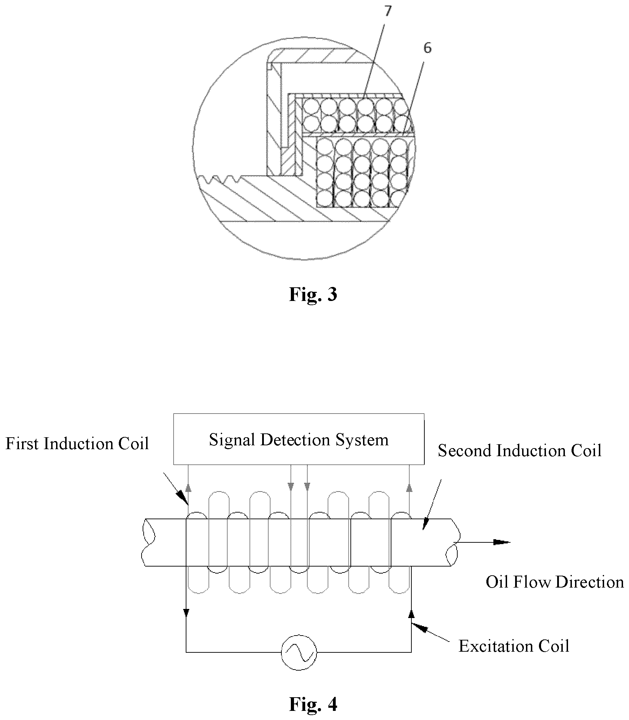

As shown, Fig. 2 is a cross-sectional structural view showing a second preferred embodiment of a device for detecting magnetic induction particles of the present disclosure. The difference between this embodiment and the above embodiment I is that, as shown in Fig. 3, a spacer sleeve 6 is arranged between the excitation coil and the induction coils, that is, the spacer sleeve is covered outside the excitation coil and the induction coils are wound on the spacer sleeve. Additionally, a shielding ring 7 is arranged outside the induction coils.

The above two technical solutions may be implemented together, or one of them may be implemented, as needed. In the present embodiment, both solutions are implemented, i.e., both the spacer sleeve and the shielding ring are arranged, which is a more preferable embodiment.

On one hand, the spacer sleeve is provided mainly for isolation of the excitation coil and the induction coils during the production and manufacture process; and on the other hand, the spacer sleeve may plays a role of a skeleton for winding the induction coils at the same time, which may improve the flatness of the wound induction coils. As a still further preferable embodiment, the spacer sleeve is made of non-magnetic materials, so as to minimizing the magnetic field loss between the induction coils and the excitation coil during the process of sensing the magnetic field disturbance generated by metal particles. It is beneficial to improve the detection precision of metal particles and thus the non-magnetic materials is used here.

The shielding ring is provided. The shielding ring is arranged outside the induction coils to isolate an external magnetic field and resist the interference of the external magnetic field, so that a detection result is more accurate and a detection effect is better.

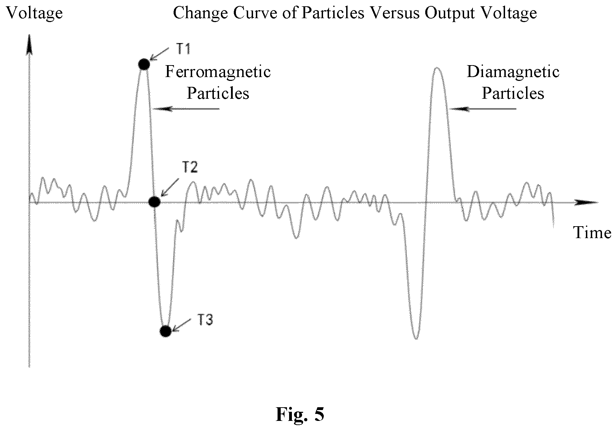

Taking the arrangement in the above embodiments as an example, the implementation principle of the device is illustrated in connection with Fig. 4 and Fig. 5 below.

An alternating magnetic field may be generated by inputting a sinusoidal alternating signal to both ends of the excitation coil; an alternating signal may be generated at both ends of the induction coil under the action of the alternating magnetic field.

Metal material can be roughly classified into diamagnetic (<1), paramagnetic (>1), and ferromagnetic (>>1), according to the magnetic permeability of the materials. Among them, the diamagnetic material weakens the magnetic field, the paramagnetic material strengthens the magnetic field, and the ferromagnetic material increases the magnetic field strength greatly. In a circuit, the reverse output ends of the two induction coils are connected and output signals from the other two ends are measured. When no metal particle passes through the inside of the excitation coil, the induced signals of the two induction coils are cancelled reversely, and the overall output of the system is zero. When metal particles (ferromagnetic materials) pass through the inside of the excitation coil from left to right, the following processes happen.

(1) During a process that the metal particles enter the first induction coil, the first induction coil is more sensitive to the change and the corresponding voltage value is increased firstly; the second induction coil changes slowly; and a rising positive voltage is outputted at both ends of the induction coils.
(2) As the metal particles approach to the middle, the second induction coil is also affected. At this time, the voltage generated by the first induction coil is balanced slowly by the voltage generated by the second induction coil, decreases gradually, and drops to 0 in the middle of the first induction coil and the second induction coil.
(3) The metal particles pass through the first induction coil and enter the second induction coil. At this time, the voltage value of the second induction coil is higher than that of the first induction coil, and a negative voltage appears, of which the voltage amplitude value increases continuously.
(4) When the particles pass through and flow out from the second induction coil, the influence on the second induction coil is weakened gradually, and the voltage amplitude value decreases gradually, and tends to zero after the particles flow out from the second induction coil a certain distance.

According to the electromagnetic induction principle, when metal particles pass through the oil pipeline from left to right, the sensor device can detect a signal similar to a sinusoidal wave, of which the amplitude value is proportional to the size of the particles, and the period is proportional to the flow velocity of the particles. Thereby, the flow velocity may be calculated.

**Embodiment III (A method for concentration detection using the device for detecting magnetic induction particles)**

The embodiment provides a method for concentration detection using the device for detecting magnetic induction particles of the above embodiment. The method includes the following steps:

S1: obtaining an output signal of the signal detection system, to acquire a change of a voltage amplitude;
S2: detecting a concentration of metal particles according to the change of the voltage amplitude;

wherein the change of the voltage amplitude includes the change of the voltage amplitude in both an amplitude value and time, i.e., a relationship between a change of the amplitude value and a moment when it happens, for example, the amplitude value at a certain point and the corresponding moment.

In an preferable embodiment, detecting the concentration of metal particles includes the following steps:

obtaining a flow velocity (v) by which metal particles pass through the induction coils;
obtaining a mass (m) of the metal particles; and calculating a concentration (c) of particles by the following equation, according to the obtained flow velocity (v) and mass (m) of the metal particles, and a time duration (t) took by the metal particles to pass through the induction coils, and utilizing a cross-sectional area (S) of the detection pipeline:

$$c = \frac{m}{v * t * S}.$$

In a more preferable embodiment, the method for calculating the flow velocity (v) of the metal particles includes the following steps:
when the metal particles passes through a group of induction coils:

recording first time and second time at which the signal processing system measures that the voltage amplitude is at the highest point and a zero-crossing point of a positive half cycle respectively, and calculating a first time difference ($\Delta T1$) between the first time and the second time, and a first corresponding length (LI) of induction coil; recording third time and fourth time at which the signal processing system measures that the voltage amplitude is at a zero-crossing point and the highest point of a negative half cycle respectively, and calculating a second time difference ($\Delta T2$) between the third time and the fourth time, and a second corresponding length (L2) of induction coil;
measuring the flow velocity (v) based on the following equation:

$$v = \frac{\dfrac{k1 * L1}{\Delta T1} + \dfrac{k2 * L2}{\Delta T2}}{2}.$$

Because there are too many detection points of the zero-crossing point in the output signal, it is often possible to cause errors in the actual sampling process. Therefore, in this method, the highest point of the positive/negative half-cycle of the signal is selected as the time recording point for the flow velocity analysis.

During the process that particles flows through the oil pipeline, the length (L) of the pipeline is constant, and may be extracted by sampling times T1, T2 and T3, where T1 is the moment when the signal passes the highest point of the positive half cycle, and T2 is the moment when the signal passes the zero point, and T3 is the moment when the signal passes the highest point of the negative half cycle, as shown in Fig. 5. The flow velocity may be obtained as:

$$v = K * \frac{L}{\Delta T}$$

Because that different factors of each oil sensor, such as, the wire (thickness and material) the number of winding turns, the interaction of the two induction coils and so on, affect the output signal of the sensor, leading to that the sensor cannot sense the middle part of the coil, a correction coefficient K is used to correct it. At the same time, an average of velocities are taken to reduce the error, by the analysis of two time periods from T1 to T2 and T2 to T3.

$$v_1 = K * \frac{L}{2 * (T_2 - T_1)}$$

$$v_2 = K * \frac{L}{2 * (T_3 - T_2)}$$

$$v = \frac{v_1 + v_2}{2}$$

where L is the total length of the induction coils passed through by the particles, and L/2 is the length of the induction coils passed through during each of the two half cycles.

The above describes the velocity calculated when the particles pass through a group of induction coils.

In the output signal, the amplitude value of the signal is related to the size of the metal particles. In a case that cylindrical metal particles pass through the inside of a spiral pipe at a constant speed, the induced electromotive force is generated as:

$$E = -4k\mu_0\mu_r n^3 VI_0 v$$

where k is the system correction coefficient, n is a density of turns of the coils (the number of turns per length unit = total turns / total length), V is the volume of the particles, and v is the flow velocity of the particles.

In the single-layer closely wound coil, the induced voltage (E) caused by the metal particles when passing through the spiral pipe induction coil is proportional to t a volume (V), magnetic permeability, and flow velocity (v) of the particles, and the third power of the v. By quantitative analysis on the output signal of the sensor, the volume and mass of the metal abrasive particles flowing through the oil pipeline may be converted. The concentration of the metal particles may be measured based on the following method, under the condition that the oil flow velocity (v) is obtained.

The cross-sectional area (S) of the pipeline is known. The concentration of the metal particles may be obtained by converting the quantity and size of metal particles passing through the coils obtained based on amplitude value of the output signal into a total mass (m), i.e., calculated by the equation below:

$$c = \frac{m}{v * t * S} (ug / m^3)$$

In a still further preferable embodiment, a frequency of obtaining the output signal of the signal detection system in S1 is once per millisecond (ms).

**Embodiment IV (A method for concentration detection using the device for detecting magnetic induction particles)**

The difference between the embodiment and the above embodiment III is that the flow velocity (v) of the embodiment applies a more preferable implementation, that is, if there are multiple groups of induction coils, the flow velocity (v) of the metal particles passing through the induction coils is an average value of the flow velocity for each group of induction coils.

For example, in S1, the flow velocity (vgn) (where n is a positive integer) of the metal particles passing through the gn[th] group of induction coils is calculated respectively, and the flow velocity (v) is an average value of the flow velocity of each group of induction coils, that is:

$$v = \frac{vg1 + vg2 + \cdots + vgn}{n}$$

The calculation method of the average value can improve the calculation precision of the flow velocity, and the calculation result is more accurate.

For example, in the device, there are two groups induction coils in total. The flow velocity measured for the first group of induction coils is vg1 and the flow velocity measured for the second group of induction coils is vg2, and thus the final flow velocity of S1 is to be calculated by the equation below:

$$v = \frac{vg1 + vg2}{2}.$$

The above embodiments are merely preferable embodiments of the present disclosure, and the scope of the present disclosure is not limited thereto. Any insubstantial changes and substitutions made by those skilled in the art based on the present disclosure should belong to the protection scope claimed by the present disclosure.

**Claims**

1. A device for detecting magnetic induction particles, **characterized by** comprising:

   a signal detection system, a detection pipeline, an excitation coil, and a positive even number of induction coils, wherein the excitation coil is connected to a signal processing system and wound on the detection pipeline; the induction coils are connected to the signal processing system respectively and wound on the excitation coil successively, and each of the induction coils is wound on the excitation coil reversely from another induction coil directly adjacent to the induction coil;
   preferably, the number of induction coils is two, four or six.

2. The device for detecting magnetic induction particles according to claim 1, **characterized in that**, the excitation coil includes two or more excitation coils wound on the detection pipeline in the same direction respectively.

3. The device for detecting magnetic induction particles according to claim 1 or claim 2, **characterized in that**, the excitation coil and/or the induction coils are wound in a manner of at least one layer.

4. The device for detecting magnetic induction particles according to any of claims 1-3, **characterized in that**, the detection pipeline is made of non-magnetic materials;
   preferably, the detection pipeline is made of stainless steel.

5. The device for detecting magnetic induction particles according to any of claims 1-4, **characterized in that**, a spacer sleeve is further disposed between the excitation coil and the induction coils;
   preferably, the spacer sleeve is made of non-magnetic materials.

6. The device for detecting magnetic induction particles according to any of claims 1-5, **characterized in that**, a shielding ring is further disposed outside the induction coils.

7. A method for concentration detection using the device for detecting magnetic induction particles according to any of claims 1-6, **characterized in that**, the method comprises:

   S1: obtaining an output signal of the signal detection system, to acquire a change of a voltage amplitude; and
   S2: detecting a concentration of metal particles according to the change of the voltage amplitude;

   preferably, detecting the concentration of metal particles comprises:

   obtaining a flow velocity v by which metal particles pass through the induction coils;
   obtaining a mass m of the metal particles; and calculating a concentration c of particles by the following equation, according to the obtained flow velocity, v, and mass, m, of the metal particles, and a time duration t took by the metal particles to pass through the induction coils, and utilizing a cross-sectional area S of the detection pipeline:

$$c = \frac{m}{v * t * S}.$$

8. The method for concentration detection according to claim 7, **characterized in that**, obtaining the flow velocity v of the metal particles comprises:
   when the metal particles pass through a group of induction coils:

   recording first time and second time at which the signal processing system measures that the voltage amplitude is at the highest point and a zero-crossing point of a positive half cycle respectively, and calculating a first time difference $\Delta T1$ between the first time and the second time, and a first corresponding length L1 of induction coil; recording third time and fourth time at which the signal processing system measures that the voltage amplitude is at a zero-crossing point and the highest point of a negative half cycle respectively, and calculating a second time difference $\Delta T2$ between the third time and the fourth time, and a second corresponding length L2 of induction coil;

measuring the flow velocity v based on the following equation:

$$v = \frac{\dfrac{k1 * L1}{\Delta T1} + \dfrac{k2 * L2}{\Delta T2}}{2}.$$

9. The method for concentration detection according to claim 7 or claim 8, **characterized in that**, when the induction coils comprises multiple groups of induction coils, the flow velocity v by which the metal particles pass through the induction coils equals an average value of flow velocities v each corresponding to one of the multiple groups of induction coils.

10. The method for concentration detection according to any of claims 7-9, **characterized in that**, a frequency of obtaining the output signal of the signal detection system in S1 is once per millisecond.

**Fig. 1**

**Fig. 2**

7

6

Fig. 3

First Induction Coil

Signal Detection System

Second Induction Coil

Oil Flow Direction

Excitation Coil

Fig. 4

Fig. 5

## INTERNATIONAL SEARCH REPORT

| International application No. |
| --- |
| **PCT/CN2018/118694** |

| **A.** | **CLASSIFICATION OF SUBJECT MATTER** |
| --- | --- |

G01N 15/06(2006.01)i

According to International Patent Classification (IPC) or to both national classification and IPC

| **B.** | **FIELDS SEARCHED** |
| --- | --- |

Minimum documentation searched (classification system followed by classification symbols)

G01N，G01R

Documentation searched other than minimum documentation to the extent that such documents are included in the fields searched

Electronic data base consulted during the international search (name of data base and, where practicable, search terms used)

CNTXT, CNABS, CNKI: 激励, 感应, 检测, 线圈, 浓度, 密度, 颗粒, 磨粒, 电磁, 磁感应, 质量, 横截面积, 时间, 速度, 流速;
VEN: exciting, induction, detect, coil, concentration, density, grain, particle, magnetic, mass, cross sectional area, time, speed

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| Y | US 5001424 A (PRODUCT RESOURCES INC.) 19 March 1991 (1991-03-19)<br>    description, column 1, line 59 to column 3, line 17, and figure 2 | 1-10 |
| Y | LIU, Xiaolin et al. "三线圈内外层电感磨粒传感器研究 (Research on Three-Coil Inner and Outer Layer Inductive Wear Particle Sensor)"<br>传感器与微系统 (Transducer and Microsystem Technologies),<br>Vol. 33, No. (11), 20 November 2014 (2014-11-20),<br>ISSN: 1000-9787,<br>    main body, parts 2 and 4 | 1-10 |
| Y | CN 105954067 A (CENTRAL SOUTH UNIVERSITY) 21 September 2016 (2016-09-21)<br>    claim 7 | 7-10 |
| Y | CN 103344535 A (GUILIN UNIVERSITY OF ELECTRONIC TECHNOLOGY) 09 October 2013 (2013-10-09)<br>    description, paragraphs 3-8, and figures 1-2 | 1-10 |
| Y | CN 103592208 A (NATIONAL UNIVERSITY OF DEFENSE TECHNOLOGY ET AL.) 19 February 2014 (2014-02-19)<br>    description, paragraphs 7-13, and figures 1-3 | 1-10 |

☑ Further documents are listed in the continuation of Box C.    ☑ See patent family annex.

| | |
| --- | --- |
| \*   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **08 January 2019** | **29 January 2019** |

| Name and mailing address of the ISA/CN | Authorized officer |
| --- | --- |
| **State Intellectual Property Office of the P. R. China**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2018/118694** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 101963570 A (SHENZHEN YATEKS OPTICAL ELECTRONIC TECHNOLOGY CO., LTD.) 02 February 2011 (2011-02-02)<br>description, paragraphs 4-21, and figures 1-2 | 1-10 |
| PX | CN 107907455 A (FATRI XIAMEN TECHNOLOGIES LTD.) 13 April 2018 (2018-04-13)<br>claims 1-10 | 1-10 |
| PX | CN 108051348 A (FATRI XIAMEN TECHNOLOGIES LTD.) 18 May 2018 (2018-05-18)<br>claims 1-10 | 1-10 |
| PY | CN 108169086 A (FATRI XIAMEN TECHNOLOGIES LTD.) 15 June 2018 (2018-06-15)<br>description, paragraphs 4-32 | 7-10 |
| PY | CN 206863240 U (UNIVERSITY OF JINAN) 09 January 2018 (2018-01-09)<br>description, paragraphs 20-24, and figures 1-2 | 1-10 |
| A | US 2015091556 A1 (ELECTRONICS AND TELECOMMUNICATIONS RESEARCH INSTITUTE) 02 April 2015 (2015-04-02)<br>entire document | 1-10 |
| A | CN 105954156 A (SHAOXING UNIVERSITY) 21 September 2016 (2016-09-21)<br>entire document | 1-10 |

Form PCT/ISA/210 (second sheet) (January 2015)

# EP 3 722 782 A1

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| US | 5001424 | A | 19 March 1991 | None | | | |
| CN | 105954067 | A | 21 September 2016 | None | | | |
| CN | 103344535 | A | 09 October 2013 | CN | 103344535 | B | 22 April 2015 |
| CN | 103592208 | A | 19 February 2014 | None | | | |
| CN | 101963570 | A | 02 February 2011 | CN | 101963570 | B | 01 August 2012 |
| CN | 107907455 | A | 13 April 2018 | None | | | |
| CN | 108051348 | A | 18 May 2018 | None | | | |
| CN | 108169086 | A | 15 June 2018 | None | | | |
| CN | 206863240 | U | 09 January 2018 | None | | | |
| US | 2015091556 | A1 | 02 April 2015 | KR | 20150036941 | A | 08 April 2015 |
| CN | 105954156 | A | 21 September 2016 | None | | | |

Form PCT/ISA/210 (patent family annex) (January 2015)